# EUROPÄISCHE PATENTSCHRIFT

(11) **EP 2 836 109 B1**
(45) Veröffentlichungstag und Bekanntmachung des Hinweises auf die Patenterteilung: **15.12.2021**
(21) Anmeldenummer: 13725894.3
(22) Anmeldetag: 10.04.2013
(51) Int. Cl.: A61B 5/00, A61B 5/11, G06K 9/00

(54) **AKUSTISCHE ERMITTLUNG DER GANGART EINES HUFTIERES**
ACOUSTIC DETERMINATION OF THE GAIT OF A HOOFED ANIMAL
DÉTERMINATION ACOUSTIQUE DE LA DÉMARCHE D'UN ANIMAL À SABOTS

(30) Priorität: 13.04.2012 DE 102012007436
(43) Veröffentlichungstag der Anmeldung: 18.02.2015
(73) Patentinhaber: FBN - Leibniz-Institut für Nutztierbiologie, 18196 Dummerstorf (DE)
(72) Erfinder: SCHÖN, Peter-Christian, 18190 Sanitz (DE); WENDLAND, Kurt, 18184 Roggentin (DE)
(74) Vertreter: Grünbaum, Annekathrin
(86) Internationale Anmeldenummer: PCT/DE2013/000184
(87) Internationale Veröffentlichungsnummer: WO 2013/152754

(56) Entgegenhaltungen:
- WO-A1-01/91640
- DE-U1-202006 017 301
- JP-A- 2000 193 520
- US-A- 4 195 643
- US-A1- 2002 107 649
- US-A1- 2010 260 011

## Beschreibung

Die Erfindung betrifft eine Vorrichtung zur akustischen Ermittlung der Gangart von Huftieren, insbesondere landwirtschaftlichen Nutztieren. Die ermittelten Werte der Gangart und die Abweichung von Standardwerten können unter anderem später der Ermittlung von beispielsweise Huferkrankungen im Frühstadium dienen.

Bei bisher bekannten Verfahren und Vorrichtungen zur Gangartermittlung von landwirtschaftlichen Nutztieren werden vor allem Drucksensoren zur Bewegungsdetektion des Tieres verwendet. Diese können entweder im Boden eingelassen sein und so die Bewegung des Tieres detektieren (US 4195643, US 20040158174A1 und M.A. Weishaupt et al., "Compensatory load redistribution of horses with induced weight-bearing forelimb lameness trotting on a treadmill", The Veterinary Journal, Vol. 171, pp. 135-146, 2006) oder an den Hufen befestigt sein und zur Auswertung die Daten mittels Funk an die Auswerteeinheit übertragen (WO 9721345).
Bei einer weiterführenden Ausgestaltung werden auch noch Lagedaten vom Kopf und des Beckens zusätzlich zu den Sensordaten der Hufe mittels Funkübertragung zur Auswertung herangezogen (WO 2008011590A2).

Eine berührungslose Methode zur Untersuchung des Ganges der Tiere ist die Bildanalyse. So kann z. B. von der Seite das Tier mittels Video oder einer Fotoserie aufgenommen werden und der Gang von einer Auswerteeinheit analysiert werden (WO 2007107986 A2). Bei einer weiteren Variante mittels Bildanalyse wird das Tier über ein durchsichtiges Laufband geführt. Dabei wird die Aufzeichnung der Schrittmuster von unten durchgeführt (JP 203250780 A).

Diese Verfahren und Vorrichtungen weisen jedoch erhebliche Nachteile auf. Bei den Methoden, die nicht berührungsfrei sind, werden die Drucksensoren großen Belastungen ausgesetzt, so dass ein Ausfall der Sensorik aufgrund von Verschleiß zu erwarten ist. Bei berührungsfreien Methoden ist dieser Nachteil nicht vorhanden. Allerdings bedarf die Bildanalyse einer rechenstarken Auswerteeinheit, um die großen Datenmengen der Kameras verarbeiten zu können, so dass nur sehr rechenstarke Auswerteeinheiten eine Echtzeitanalyse vornehmen können. Desweiteren muss bei Bildverarbeitungsverfahren auch die Ausleuchtung des zu analysierenden Stands passen, so dass der Gegenstand sich gut vom Hintergrund abhebt und das Bild somit gut charakterisierbar ist.

In der US PS 2002/0107649 ist ein System, eine Vorrichtung und eine Methode zur Ermittlung der Gangart von Personen beschrieben. Damit soll die Schrittzahl bestimmter Personen und darauf aufbauend die zurückgelegte Wegstrecke, bzw. Entfernung von einem Standort bestimmt werden können. Bei der bekannten Aufnahme von Tönen mittels Mikrofon und Bewertung der Signalspitzen als Schritte ist häufig keine genaue Schrittbestimmung möglich, da Hintergrundgeräusche störend wirken. Weiterhin ist nicht ermittelbar, welches Gangbild, wie Laufen, Wandern, Treppensteigen usw. vorliegt. Weshalb z. B. keine genaue zurückgelegte Weglänge aufgrund der Schrittzahl ermittelt werden kann. Zur Verbesserung der Ganganalyse wird in dieser US PS in Anspruch 1 beansprucht, in einem System zur Erfassung der Schrittgeräusche mit einem Mikrofon nur einen bestimmten Frequenzbereich auszuwerten. Dabei wird gemäß Anspruch 2 die Dauer und Häufigkeit der Intensität eines Signals im Bereich weniger oder gleich 100 Hz bewertet. In der Vorrichtung wird zur Frequenzauswahl ein Low-Passfilter zwischen Mikrofon und Converter geschaltet. Weiterhin werden mit Datenspeichermitteln der Vorrichtung Gangartmuster gespeichert. Eine Analyseeinheit vergleicht die eingehenden Daten mit diesen Gangartmustern auf Übereinstimmung, damit nur die Schritte dieser Person erfasst werden. Durch externe Eingabe der Größe der Person, bzw. deren Schrittlänge kann mit der Anzahl der erfassten Schritte die zurückgelegte Wegstrecke berechnet werden.

Spezielle Merkmale der Gangart, wie unterschiedliche Auftrittstärke, ungleiche Schrittlänge usw., die durch Fehlstellungen oder Erkrankungen bedingt sind, können mit dieser Erfindung nicht erfasst werden.
Es wird auch nur ein bestimmter Frequenzbereich der Trittgeräusche aufgenommen und darin nur die Signalspitzen als Schritte bewertet, so dass weitergehende Informationen nicht gewonnen werden können.
Zur genaueren Erfassung und Bewertung der Gangart von Nutztieren, wie Huftiere, ist die Erfindung nicht geeignet.

In der JP 2000 193520 A wird eine Vorrichtung zur Identifizierung von Pferden durch einen an diesem befestigten Sensor vorgeschlagen. Dieser misst dreidimensional die Amplitude und Frequenz der Bewegung. Die Werte werden an eine Dateneinheit übertragen, welche einen Vergleich mit den vorhandenen Daten durchführt.
Eine genaue, differenzierte Erfassung des Trittmusters des Pferdes ist damit nicht möglich.

In der US 2010/0260011 A1 wird ein System, Verfahren und Vorrichtung zur Erfassung von seismischen Daten und deren Auswertung vorgeschlagen. Diese dienen der Überwachung der Annäherung von Personen, Tieren oder Fahrzeugen. Als Signalgeber werden Schwingungssensoren, bzw. Geophone eingesetzt. Diese Erfindung ermöglicht eine Messung des Schwingungs-Zeitverlaufes und dient zur Erfassung und Klassifizierung eines allgemeinen Gangmusters. Genauerer Werte zur Ermittlung der Gangarten, wie der einzelnen Varianten der Lahmheit von Tieren, sind hiermit nicht ermittelbar.

Das betrifft auch die Erfindung gemäß der WO 01/91640 A1. Diese beschreibt ein Verfahren und eine Vorrichtung zur Ermittlung der Lahmheit von Tieren. Es wird eine mehrteilige Bodenplatte vorgeschlagen, an der Lastzellen befestigt sind. Die sich ergebenden Lastverteilungen werden erfasst und aufbereitet. Lastmessungen können nur allgemeine Flächenbelastungen und nur begrenzt die Dynamik erfassen. Eine differenzierte Erfassung der einzelnen Auftritte ist nicht möglich.
Verfahren und Vorrichtungen die Drucksensoren verwenden besitzen weitere Nachteile, wie einem größeren Verschleiß und bei mehreren Drucksensoren die Verarbeitung einer großen Datenmenge.

Gemäß der DE 20 2006 017 301 U1 (siehe auch EP 2097 002 B1) wird zur Erfassung des Trittprofils vorgeschlagen, um eine geneigt angeordnetes Trittplatte ein Laufband anzuordnen. Diese ist einseitig frei gelagert und gegenüber der Lagerung ist eine Abstützung mit einem Beschleunigungssensor installiert, der die Druckimpulse der Auftritte erfasst. Diese Impulse werden einer Auswerteeinheit zugeführt.
Diese Vorrichtung ist bedingt durch ein Laufband und eine bewegliche Lagerung, sowie speziell gelagerten Beschleunigungssensor technisch aufwendig. Der eingesetzte Beschleunigungssensor misst die durch die Auftritte erzeugten Schwingungen. Diese sind aber je nach Entfernung des Auftritts verschieden, was unterschiedliche Signale bewirkt. Für eine genauere Messung wäre eine Vielzahl von Sensoren verteilt auf der Trittplatte erforderlich.
Weiterhin sind Trittwechsel des Tieres auf dem Laufband wahrscheinlich, was ebenfalls die Messwerte beeinträchtigt. Die Bewegung des Tieres auf dem Laufband zur Messwerterfassung benötigt einen größeren Zeitraum. Der Zeitaufwand für die Erfassung einer Tierherde ist deshalb sehr hoch.
In der Schrift wird auch der mögliche Einsatz von akustischen Sensoren erwähnt. Konkrete Ausführungen sind dazu aber nicht enthalten.

### Aufgabe der Erfindung

Daher liegt der Erfindung die Aufgabe zugrunde, eine Vorrichtung der eingangs genannten Art bereitzustellen, bei dem eine gegebene Gangart von Huftieren, speziell landwirtschaftlichen Nutztieren, sehr genau ermittelt wird. Hierbei soll vor allem nicht zwischen den unterschiedlichen normalen Gangarten differenziert werden (Schritt, Galopp etc.), vielmehr sollen unnatürliche Weisen einer Gangart erkannt werden. Insbesondere sollen verschieden Formen des Lahmens der Huftiere differenziert erfasst und entsprechende Daten aufbereitet werden.
Des Weiteren soll die Erfassung einfach in der Anwendung und innerhalb kürzester Zeit abgeschlossen sein, so dass das Verfahren und die Vorrichtung bei großen Tierbeständen effektiv anwendbar sind. Zudem soll die Vorrichtung technisch einfach ausgelegt und transportabel sein, damit diese auch auf Bauernhöfen und verschiedenen Orten im Außenbereich betrieben werden kann.

Gemäß einem nicht erfindungsgemäßen Verfahren, gemäß dem bei der akustischen Aufnahme mit einer Vorrichtung mit zumindest einem Schallsensor (5) am Untergrundbereich (3), dessen Länge mindestens 4 Schrittlängen bis maximal der doppelten Körperlänge des Huftieres beträgt, um eine Änderung der Gangart/des Trittwechsels zu vermeiden. Der/die Schallsensor/en (5) erfassen einen Frequenzbereich von mindestens 20 Hz bis 5 kHz und die analogen Ausgangssignale werden an eine Signalaufbereitungseinheit (7) weitergeleitet und von dieser digitalisiert. Von der Signalaufbereitungseinheit (7) werden mindestens die spezifischen Parameter Zeitablauf und Verlauf der Auftritte, Frequenzspektrum und deren Verlauf, sowie das Schallpegelspektrum in mindestens einen Parametervektor konvertiert. Der/diese Parametervektor/en wird/werden an eine Auswertungseinheit (8) weitergeleitet und dort klassifiziert.

Wird das landwirtschaftliche Nutztier/Huftier (1) über einen festen Untergrundbereich mit einer geringen Dämpfung des Körperschalls getrieben, erzeugen die Hufe beim Auftreten detektierfähige Geräusche. Diese werden mit mindestens einem Schallsensor aufgenommen, der ausgangseitig mit einer Signalaufbereitungseinheit verbunden ist. So kann in der Signalaufbereitungseinheit das analoge Eingangssignal digitalisiert und in einen Parametervektor umgesetzt werden. In der Auswerteeinheit wird dieser zur Ermittlung der Gangart verwendet. Um eine akustische Trittmustererkennung überhaupt zu ermöglichen, muss das Tier mindestens vier Tritte auf dem Untergrundbereich machen. Folglich muss der Untergrundbereich eine entsprechende Mindestlänge besitzen.

Die mit der Erfindung erzielten Vorteile bestehen insbesondere darin, dass eine genaue kontaktlose Analyse der Gangart möglich ist und so kein Verschleiß der Sensorik auftritt. Die aufgenommenen Daten können auf Grund der geringen Datenmengen und des einfachen Auswerteverfahrens mit rechenschwachen Auswerteeinheiten wie Mikroprozessoren in Echtzeit ausgewertet werden, so dass die Erfindung in Betrieben mit großen Tierbeständen eingesetzt werden kann. Zudem sind der Aufbau und die Anwendung sehr einfach gehalten, was eine mobile Anwendung des Verfahrens ermöglicht und somit in verschiedenen Großbetrieben und auch Kleinbetrieben einsetzbar ist.

Ein weiterer wichtiger Aspekt für eine mobile Anwendung ist, dass im Vergleich zur Bildanalyse mit dem akustischen Verfahren geringere Fehlerraten bei geringerem Aufwand erzielt werden, die durch weitere Störquellen und spezifischen Eigenschaften der jeweiligen Umgebung verursacht werden.

Gemäß einer bevorzugten Ausführung des nicht erfindungsgemäßen Verfahrens werden die analogen Signale zum Zeitablauf der Auftritte von der Signalaufbereitungseinheit (7) digitalisiert und nach der Dauer des Einzelauftritts, der Zeit zwischen zwei Auftritte, der Gesamtzeit und des Frequenzmusters als Parametervektor aufbereitet.

Es werden die analogen Signale zum Verlauf der Auftritte von der Signalaufbereitungseinheit (7) digitalisiert und nach dem Ablauf und der Größe Lautstärke über der Zeit als Parametervektor aufbereitet.

Es werden die analogen Signale zum Frequenzspektrum und deren Verlauf von der Signalaufbereitungseinheit (7) digitalisiert und nach der Anzahl und dem Abstand der Frequenzbänder, sowie dem Verlauf der Frequenzbänder über der Zeit als Parametervektor aufbereitet.

Gemäß einer weiteren bevorzugten Ausführung des nicht erfindungsgemäßen Verfahrens werden die analogen Signale zum Schallpegelspektrum von der Signalaufbereitungseinheit (7) digitalisiert und nach der maximalen, sowie Anzahl und dem Abstand der Peakfrequenzen, sowie der Frequenz und Amplitude der Peakfrequenzen als Parametervektor aufbereitet.

Die speziellen Varianten zur Aufbereitung der digitalisierten Daten sind bevorzugt auch miteinander kombinierbar. Das ermöglicht eine sehr genaue Bewertung der einzelnen Merkmale der Gangart.

Es werden der Parametervektor/die Parametervektoren für ein allgemeines/standardisiertes Trittmuster eines Nutztieres (1) oder eine Tierart/Tiergattung bereitgestellt.

Damit wird eine Klassifikation der Parameter für eine Tierart/Tiergattung und werden Standardwerte für ein konkretes, gesundes Nutztier (1) ermöglicht. Diese Verfahrensweise vereinfacht erheblich eine spätere Nutzung der Daten, z. B. für eine Erkennung von Verletzungen bzw. Erkrankungen am Huf oder Bewegungsapparat eines Tieres durch Vergleich der aktuell erfassten Parametervektoren mit Standardwerten. Da jedes Nutztier (1) auch im gesunden Zustand geringfügige Abweichungen in den Parametern gegenüber verwandten Nutztieren (1), also individuelle Werte, besitzt, können Abweichungen von den eigenen normalen Parametern sehr genau ermittelt werden.

Es erfolgt das Klassifizieren und Konvertieren unter Anwendung eines neuronalen Netzes und es wird durch einen Schritt des Trainierens des neuronalen Netzes mittels vorgegebener Trainingsauftretgeräusche eine Klassifikation realisiert.

Die erfindungsgemäße Vorrichtung zur Durchführung des Verfahrens gemäß Anspruch 1 besitzt eine stationäre Wegstrecke mit einem gleichförmigen Untergrundbereich (3) mit mindestens 4 Schrittlängen, bzw. einer Länge für je einen Einzelauftritt der vier Hufe (2), bis maximal der doppelten Körperlänge des Huftieres, um eine Änderung der Gangart/des Trittwechsel zu vermeiden. Direkt an dem Untergrundbereich (3) ist mindestens ein Schallsensor (5) für einen Frequenzbereich von 20 Hz bis 5 kHz zur Aufnahme der Auftretgeräusche (4) angeordnet, der/die mit einer Signalaufbereitungseinheit (7) zur Digitalisierung der Ausgangssignale der Schallsensoren (5) verbunden ist/sind. Die Signalaufbereitungseinheit (7) konvertiert die spezifischen Parameter Zeitablauf und Verlauf der Auftritte, Frequenzspektrum und deren Verlauf, sowie das Schallpegelspektrum in mindestens einen Parametervektor. Eine Auswerteeinheit (8) ist mit dieser verbunden, welche den Parametervektor klassifiziert und die Ähnlichkeit mit zuvor aufgenommenen Parametervektoren bestimmt.

Ein Vorteil der Vorrichtung besteht darin, dass landwirtschaftliche Großtiere wie z. B. Pferde, Rinder, Schweine, Schafe oder Ziegen, untersucht werden können, da sie aufgrund ihres Eigengewichts beim Auftreten Geräusche erzeugen, die laut genug sind, um sich vom Hintergrundlärm abzusetzen.
Eine wesentlich größere Wegstrecke des Untergrundbereiches (3) als die 4 Schrittlängen vermeidet,. dass eine Änderung der Gangart/Trittwechsel im Übergangsbereich erfolgt, was die erfassten Werte verfälschen könnte.

In einer vorteilhaften Weiterbildung der Erfindung nach Anspruch 2 besteht der Untergrundbereich (3) aus einer harten, ebenen Fläche aus Metall, Kunststoff oder festem Mineral. Die zu- und abführenden Flächen besitzen eine geringe Steigung, so dass kein Trittwechsel des Nutztieres (1) erfolgt. Die Fläche ist auf einem Rahmen befestigt, auf dem seitliche Geländer angeordnet sind und die Einheit ist transportabel ausgebildet. Besteht der Untergrundbereich (3) aus einer harten, ebenen Fläche aus Metall, Kunststoff oder festem Mineral, werden laute charakteristische Geräusche beim Auftreten der Hufe erzielt. Durch den einfachen Aufbau ist die Vorrichtung robust und kann zu Stallanlagen oder Melkständen auf der Weide transportiert und dort genutzt werden.

Gemäß Anspruch 3 sind mehrere Schallsensoren (5) angeordnet und/oder Schallsensoren (5) als Körperschallmikrofone am Untergrundbereich (3) befestigt. So können zur Aufzeichnung der Trittgeräusche bei der Verwendung von Luftmikrofonen unter anderen räumlichen Störungen wie z. B. andere lokale Störgeräusche aber auch räumliche Verdeckung von Mitarbeitern und anderer Nutztiere oder unvorteilhafter Aufstellung kompensiert werden. Hingegen können stattdessen an der Unterseite des harten Untergrundbereiches (3) Körperschallmikrofone verwendet werden, so dass die oben genannten Problematiken bauartbedingt inhärent vermieden werden.

Gemäß einer bevorzugten Ausbildung nach Anspruch 4 umfasst die Signalaufbereitungseinheit (7) und die Auswerteeinheit (8) mindestens einen Mikroprozessor oder einen Computer mit Soundkarte (6). Es ist vorteilhaft, als Signalaufbereitungs- und Auswerteeinheit zumindest einen Mikroprozessor oder einen Computer mit Soundkarte zu benutzen um eine Flexibilität der Signalverarbeitungs- und Auswertemethodik zu gewährleisten.

Gemäß Anspruch 5 weist die Signalaufarbeitungseinheit (7) eine Vorrichtung zur Pufferung eines Signals, eine Vorrichtung zur Fensterung eines Signals und eine Vorrichtung zur Fourier-Transformation eines Signals auf.

Gemäß einer bevorzugten Ausbildung nach Anspruch 6 ist die Auswerteeinheit (8) an die Signalaufarbeitungseinheit (7) angeschlossen und umfasst ein neuronales Netz, welches bevorzugt als Multi-Layer-Perzeptron oder Kohonen Netz ausgebildet ist.
Das neuronale Netz ermöglicht nach dem Anlernen mittels der Parametervektoren von kranken und gesunden Tieren eine selbständige Klassifizierung. Das Kohonen Netz bieten die Möglichkeit die Struktur in den Daten topografisch sichtbar zu machen.

### Ausführungsbeispiele

Im Folgenden wird die Erfindung anhand einiger Ausführungsbeispiele mit Bezug auf die beiliegenden Zeichnungen und Diagramme näher erläutert. Darin zeigen:
Abb. 1 eine schematische Darstellung einer erfindungsgemäßen Vorrichtung

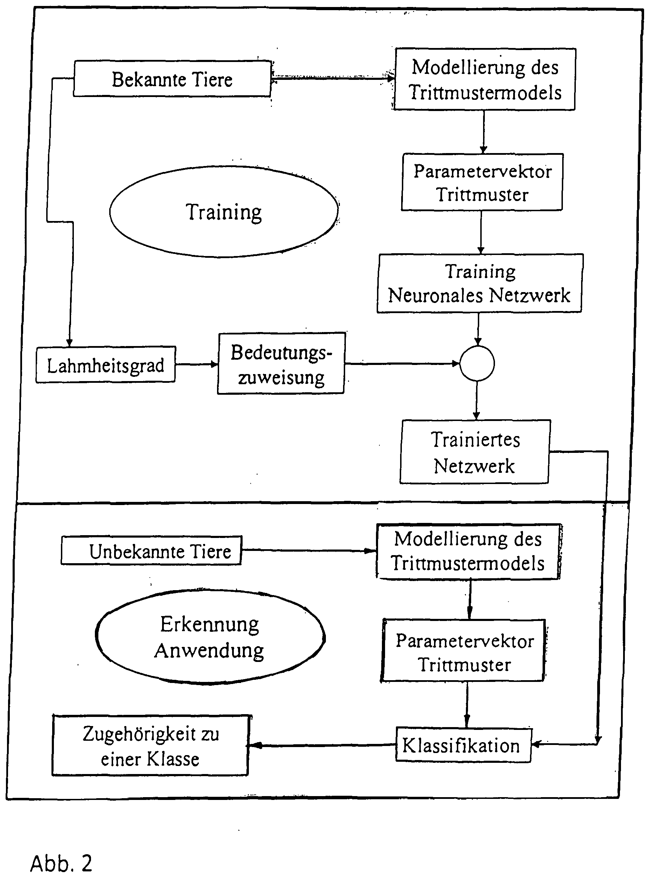

Abb. 2 Blockschaltbild zur schematischen Darstellung der Arbeitsweise der Signalaufbereitungs- und Auswerteeinheit mit einem neuronalen Netzwerk,
Abb. 3 ein Diagramm mit Parameter aus dem Zeitsignal,
Abb. 4 ein Diagramm des Powerspektrums eines Einzelauftritts,
Abb. 5 Einzelparameter eines Parametervektors und
Abb. 6 und Abb. 7 Anordnung der Parameter im dreidimensionalen Raum.

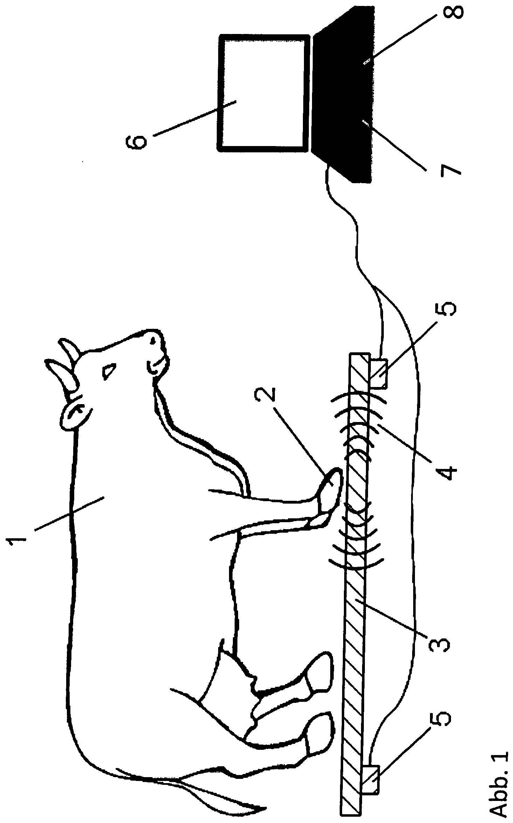

Die Vorrichtung besteht gemäß Abbildung 1 aus einer geriffelten Stahlblechplatte als fester, ebener Untergrundbereich 3. Die Abmessungen betragen für die Erfassung der Trittmuster für große Huftiere, z. B. Rinder, ca. 5 m Länge, 2 m Breite und 5 mm Dicke. Die Platte ist auf einem nicht dargestellten Rahmen befestigt, auf dem auch seitliche Geländer angeordnet sind. Die Einheit ist transportabel ausgebildet und kann somit für die Messung zum Standort der Tiere, z. B. zu einem Melkkarussell transportiert und dort am Laufgang aufgestellt werden.

Unter der Stahlblechplatte ist an jedem Ende ein Schallsensor 5 für die Messung der Schallwellen der Auftretgeräusche 4 in Form von Körperschallwellen der Stahlblechplatte befestigt. Die Schallsensoren 5 sind über ein Kabel mit einem Computer 6 mit einer Soundkarte verbunden. In dem Computer 6 sind weiterhin eine Signalaufbereitungseinheit 7 und eine Auswerteeinheit 8 als Steckkarten mit speziellem Programm angeordnet.

Zur Nutzung der Vorrichtung werden die Nutztiere 1 mit Abstand nacheinander über den Untergrundbereich 3 getrieben. Die dabei durch die Hufe 2 erzeugten Schallwellen 4 der Auftretgeräusche in der Stahlblechplatte werden von den Schallsensoren 5 erfasst und an die Signalaufbereitungseinheit 7 im PC weitergeleitet.

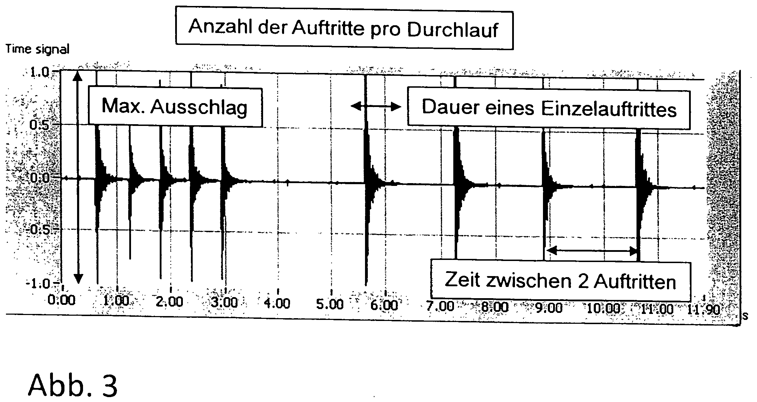

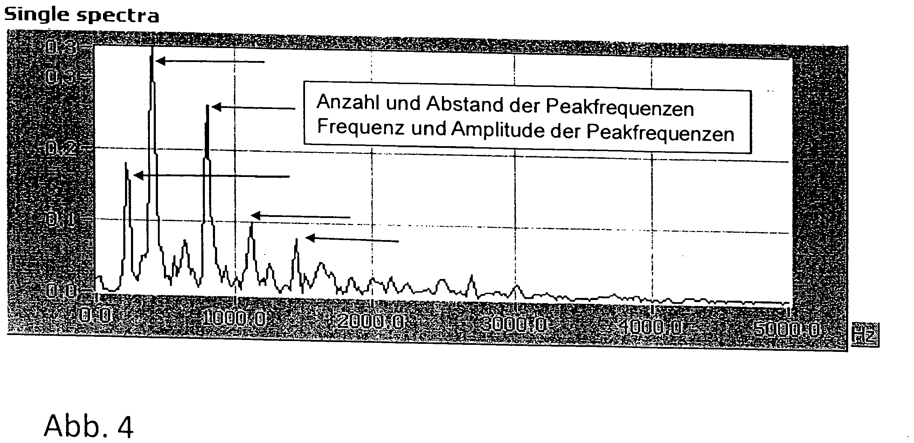

Als Beispiele für die möglichen zu erfassenden Signale zeigt die Abb. 3 als Diagramm das Eingangssignal der aufgenommenen Schallstärke der einzelnen Auftritte der Hufe 2 über der Zeit in Sekunden. Die Abb. 4 zeigt als Diagramm das Eingangssignal des Powerspektrums eines Einzelauftritts in der Amplitude über der Frequenz.

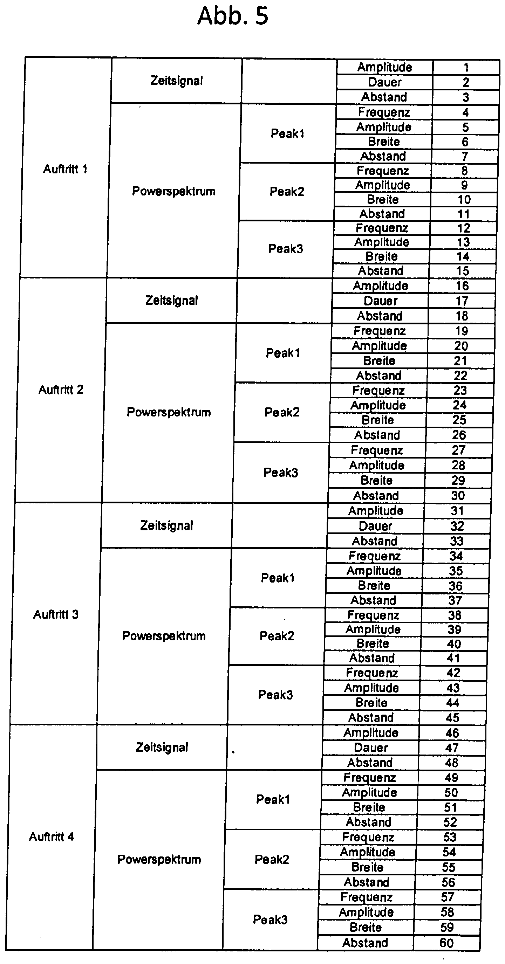

Die Signalaufbereitungseinheit 7 digitalisiert diese Signale und berechnet daraus Parametervektoren. Die Parameter werden, wie aufgezeigt, aus dem Zeitsignal, dem Frequenzspektrum, den Einzelspektren der Auftritte entnommen. Neben den dargestellten Parametern sind noch weitere Parameter denkbar. Die Sinnhaftigkeit der Parameter kann getestet werden, indem Trittmuster von gesunden und kranken Tieren aufgenommen werden und die Parameter in ihrem Mittelwert und ihrer Varianz verglichen werden. Diese Methode hat aber den Nachteil, dass Abhängigkeiten zwischen den Parametern nicht berücksichtigt werden. Eine andere Vorgehensweise ist die Behandlung der Parameter mit einer Faktorenanalyse oder Hauptkomponentenanalyse. Diese Verfahren finden die Parameter heraus, die für eine Klassifikation ausschlaggebend sind. Ein erfindungsgemäß zur Anwendung kommender Parametervektor ist in Abb. 5 dargestellt. Dieser Parametervektor hat dann eine Dimension von 60.

Aus bestimmten Parametervektoren wird ein Modell der Auftretgeräusche abgeleitet. In der Auswerteeinheit 8 werden diese mit dem Trittmustermodell verglichen, dass der Nutztierart entspricht oder aus einer früheren Messung des gleichen Tieres vorliegt. Werden Abweichen über eine bestimmte Größe ermittelt, kann das Nutztier 1 für eine genauere Prüfung ausgesondert werden.

Eine vorteilhafte Ausgestaltung der Vorrichtung weist ein neuronales Netzwerk auf, das beispielsweise als Kohonen-Netzwerkstruktur oder Perzeptronstruktur ausgestaltet ist. Anhand der Parametervektoren kann das neuronale Netz den Grad der Ähnlichkeit zu bereits zuvor aufgenommenen Parametervektoren bestimmen und eine Klassifizierung vornehmen. Aufgrund des neuronalen Netzes lässt sich der Rechenaufwand reduzieren, da zur Klassifikation des Parametervektors keine komplizierten Algorithmen notwendig sind, welches eine Vereinfachung der Auswerteeinheit mit sich bringt.

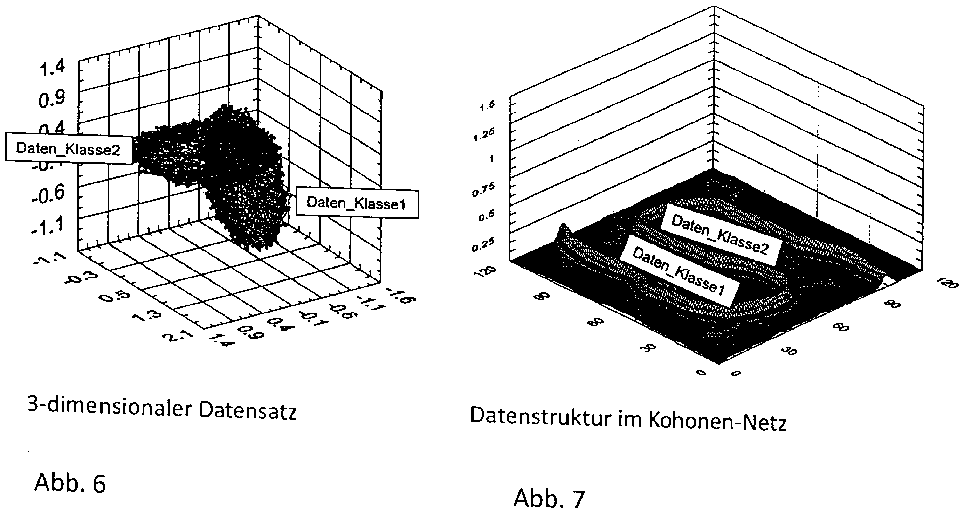

Kohonen Netze als eine spezielle Art von neuronalen Netzwerken bieten die Möglichkeit die Struktur in den Daten topografisch sichtbar zu machen. Dieses ist in den Abbildungen 6 und 7 beispielhaft dargestellt, wobei die Darstellungsweise nicht auf 3 Dimensionen beschränkt ist.

Die Erfindung ist nicht auf die vorstehend beschriebenen Ausführungsbeispiele beschränkt.

### Bezugszeichenliste

- 1: Nutztier/Huftier
- 2: Hufe
- 3: Untergrundbereich
- 4: Schallwellen der Auftretgeräusche
- 5: Schallsensor
- 6: Computer mit Soundkarte
- 7: Signalaufbereitungseinheit
- 8: Auswerteeinheit

## Patentansprüche

1. Vorrichtung zur Durchführung eines Verfahrens zur akustischen Ermittlung einer Gangart
eines Huftieres, aufweisend
einen Untergrundbereich (3) mit geringer Dämpfung des Körperschalls und eine Wegstrecke länger als für je einen Einzelauftritt der vier Hufe (2), wobei an dem Untergrundbereich (3) mindestens ein Schallsensor (5) zur Aufnahme der Auftretgeräusche (4) angeordnet ist und nachgeordnet eine Signalaufbereitungseinheit (7) sowie eine Auswerteeinheit (8) mit dem mindestens einen Schallsensor verbunden sind und wobei die Signalaufbereitungseinheit (7) dafür ausgebildet ist spezifische Parameter eines Zeitsignals, und von Powerspektren der zumindest vier Einzelauftritte in mindestens einen Parametervektor zu konvertieren, wobei von den Powerspektren für die Konvertierung des Parametervektors die spezifischen Parameter Frequenz, Amplitude, Abstand und Breite dreier Peaks verwendet werden und wobei von dem Zeitsignal die spezifischen Parameter Amplitude, Dauer eines Einzelauftritts und Abstand zwischen zwei Auftritten verwendet werden, und wobei die Auswerteeinheit (8) dafür ausgebildet ist, dass der mindestens eine Parametervektor klassifiziert und der Grad der Ähnlichkeit mit zuvor aufgenommenen Parametervektoren bestimmt wird.

2. Vorrichtung nach Anspruch 1, **dadurch gekennzeichnet, dass** der Untergrundbereich (3) aus einer harten, ebenen Fläche aus Metall, Kunststoff oder festem Mineral besteht und die zu- und abführenden Flächen eine geringe Steigung besitzen, so dass kein Trittwechsel des Nutztieres (2) erfolgt.

3. Vorrichtung nach einem der vorhergehenden Ansprüche, **dadurch gekennzeichnet, dass** mehrere Schallsensoren (5) angeordnet und/oder Schallsensoren (5) als Körperschallmikrofone am Untergrundbereich (3) befestigt sind.

4. Vorrichtung nach einem der vorhergehenden Ansprüche, **dadurch gekennzeichnet, dass** die Signalaufbereitungseinheit (7) und die Auswerteeinheit (8) mindestens einen Mikroprozessor oder einen Computer mit Soundkarte (6) umfassen.

5. Vorrichtung nach einem der vorhergehenden Ansprüche, **dadurch gekennzeichnet, dass** die Signalaufarbeitungseinheit (7) eine Vorrichtung zur Pufferung eines Signals, eine Vorrichtung zur Fensterung eines Signals und eine Vorrichtung zur Fourier-Transformation eines Signals aufweist.

6. Vorrichtung nach einem der vorhergehenden Ansprüche, **dadurch gekennzeichnet, dass** die Auswerteeinheit (8) an die Signalaufarbeitungseinheit (7) angeschlossen ist und ein neuronales Netz umfasst, welches bevorzugt als Multi-Layer-Perzeptron oder Kohonen Netz ausgebildet ist.

7. Vorrichtung nach einem der vorhergehenden Ansprüche, **dadurch gekennzeichnet**, so eingerichtet zu sein, dass die analogen Signale zum Verlauf der Auftritte von der Signalaufbereitungseinheit (7) digitalisiert und nach dem Ablauf und der Größe Lautstärke über der Zeit als Parametervektor aufbereitet werden.

8. Vorrichtung nach einem der vorhergehenden Ansprüche, **dadurch gekennzeichnet**, so eingerichtet zu sein, dass die analogen Signale zum Schallpegelspektrum von der Signalaufbereitungseinheit (7) digitalisiert und nach der maximalen Peakfrequenz, sowie Anzahl und dem Abstand der Peakfrequenzen, sowie der Frequenz und Amplitude der Peakfrequenzen als Parametervektor aufbereitet werden.

9. Vorrichtung nach einem der vorhergehenden Ansprüche, **dadurch gekennzeichnet**, so eingerichtet zu sein, dass das Klassifizieren und Konvertieren unter Anwendung eines neuronalen Netzes erfolgt und durch einen Schritt des Trainierens des neuronalen Netzes mittels vorgegebener Trainingsauftretgeräusche eine Klassifikation realisiert wird.

## Claims

1. Apparatus for carrying out a method for acoustically determining a gait of a hoofed animal, having
a ground area (3) with low attenuation of structure-borne sound, and a route longer than for a single step of each of the four hooves (2), wherein at least one sound sensor (5) for recording the step sounds (4) is arranged on the ground area (3) and, downstream, a signal conditioning unit (7) and an evaluation unit (8) are connected to the at least one sound sensor, and wherein the signal conditioning unit (7) is configured to convert specific parameters of a time signal and of power spectra of the at least four single steps into at least one parameter vector, wherein from the power spectra the specific parameters frequency, amplitude, interval and width of three peaks are used to convert the parameter vector and wherein from the time signal the specific parameters amplitude, duration of a single step and interval between two steps are used, and wherein the evaluation unit (8) is configured to classify the at least one parameter vector and to specify the degree of similarity with previously recorded parameter vectors.

2. Apparatus according to Claim 1, **characterized in that** the ground area (3) consists of a hard even surface made of metal, plastic or solid mineral and the admitting and discharging surfaces have a slight gradient, which means that no change of step in the farm animal (2) takes place.

3. Apparatus according to either of the preceding claims, **characterized in that** multiple sound sensors (5) are arranged and/or sound sensors (5) are secured to the ground area (3) as structure-borne sound microphones.

4. Apparatus according to one of the preceding claims, **characterized in that** the signal conditioning unit (7) and the evaluation unit (8) comprise at least one microprocessor or a computer with a sound card (6).

5. Apparatus according to one of the preceding claims, **characterized in that** the signal conditioning unit (7) has an apparatus for buffering a signal, an apparatus for windowing a signal and an apparatus for Fourier-transforming a signal.

6. Apparatus according to one of the preceding claims, **characterized in that** the evaluation unit (8) is connected to the signal conditioning unit (7) and comprises a neural network that is preferably in the form of a multilayer perceptron or Kohonen network.

7. Apparatus according to one of the preceding claims, **characterized in that** it is designed such that the analogue signals pertaining to the progression of the steps are digitized by the signal conditioning unit (7) and are conditioned as a parameter vector according to the sequence and the quantity loudness over time.

8. Apparatus according to one of the preceding claims, **characterized in that** it is designed such that the analogue signals pertaining to the sound level spectrum are digitized by the signal conditioning unit (7) and are conditioned as a parameter vector according to the maximum peak frequency, and the number and interval of the peak frequencies, and the frequency and amplitude of the peak frequencies.

9. Apparatus according to one of the preceding claims, **characterized in that** it is designed such that the classification and conversion take place using a neural network, and a step of training the neural network by means of predefined training step sounds is used to provide a classification.

## Revendications

1. Dispositif permettant d'effectuer un procédé de détermination acoustique d'une allure d'un ongulé, présentant une zone de support (3) ayant un faible amortissement du bruit de structure et un parcours plus long que pour respectivement un pas individuel des quatre sabots (2), au moins un capteur acoustique (5) étant disposé au niveau de la zone de support (3) pour enregistrer les bruits de pas (4), et en aval, une unité de traitement de signal (7) ainsi qu'une unité d'évaluation (8) étant reliées audit au moins un capteur acoustique, et l'unité de traitement de signal (7) étant réalisée pour convertir des paramètres spécifiques d'un signal de temps et de spectres de puissance des au moins quatre pas individuels en au moins un vecteur de paramètres, dans lequel, parmi les spectres de puissance, les paramètres spécifiques de fréquence, d'amplitude, de distance et de largeur de trois crêtes sont utilisés pour la conversion du vecteur de paramètres, et dans lequel, du signal de temps, les paramètres spécifiques d'amplitude, de durée d'un pas individuel et de distance entre deux pas sont utilisés, et l'unité d'évaluation (8) étant réalisée pour que ledit au moins un vecteur de paramètres soit classifié et le degré de similitude avec des vecteurs de paramètres enregistrés précédemment soit déterminé.

2. Dispositif selon la revendication 1, **caractérisé en ce que** la zone de support (3) est composée d'une surface dure et plane en métal, en matière plastique ou en minéral solide, et les surfaces d'accès et d'évacuation possèdent une faible pente de sorte qu'aucun changement de pas de l'animal de ferme (2) n'a lieu.

3. Dispositif selon l'une quelconque des revendications précédentes, **caractérisé en ce que** plusieurs capteurs acoustiques (5) sont disposés et/ou des capteurs acoustiques (5) sont fixés au niveau de la zone de support (3) comme des microphones de bruit de structure.

4. Dispositif selon l'une quelconque des revendications précédentes, **caractérisé en ce que** l'unité de traitement de signal (7) et l'unité d'évaluation (8) comprennent au moins un microprocesseur ou un ordinateur doté d'une carte son (6) .

5. Dispositif selon l'une quelconque des revendications précédentes, **caractérisé en ce que** l'unité de traitement de signal (7) présente un dispositif pour mettre un signal en mémoire tampon, un dispositif pour fenêtrer un signal et un dispositif pour effectuer une transformation de Fourier sur un signal.

6. Dispositif selon l'une quelconque des revendications précédentes, **caractérisé en ce que** l'unité d'évaluation (8) est connectée à l'unité de traitement de signal (7) et comprend un réseau neuronal qui est réalisé de préférence sous la forme d'un perceptron multicouche ou d'un réseau de Kohonen.

7. Dispositif selon l'une quelconque des revendications précédentes, **caractérisé en ce qu'**il est aménagé pour que les signaux analogiques concernant l'évolution des pas soient numérisés par l'unité de traitement de signal (7), et soient traités sous forme de vecteur de paramètres selon l'évolution et la grandeur de volume sonore dans le temps.

8. Dispositif selon l'une quelconque des revendications précédentes, **caractérisé en ce qu'**il est aménagé pour que les signaux analogiques concernant le spectre de niveau sonore soient numérisés par l'unité de traitement de signal (7), et soient traités sous forme de vecteur de paramètres selon la fréquence de crête maximale, ainsi que selon le nombre et la distance des fréquences de crête, ainsi que selon la fréquence et l'amplitude des fréquences de crête.

9. Dispositif selon l'une quelconque des revendications précédentes, **caractérisé en ce qu'**il est aménagé pour que la classification et la conversion soient effectuées par l'application d'un réseau neuronal, et pour qu'une classification soit réalisée par une étape d'entraînement du réseau neuronal au moyen de bruits de pas d'entraînement prédéfinis.
